# EUROPEAN PATENT SPECIFICATION

(11) **EP 0 530 517 B1**
(45) Date of publication and mention of the grant of the patent: **03.06.1998**
(21) Application number: 92113221.3
(22) Date of filing: 03.08.1992
(51) Int. Cl.: C08J 3/28, A61L 15/60, C08F 20/06

(54) **Method of treating water-insoluble superabsorbent materials**
Verfahren zur Behandlung von wasserunlöslichen Superabsorbensmaterialien
Procédé de traitement de matériaux super-absorbants insolubles dans l'eau

(30) Priority: 15.08.1991 US 745319
(43) Date of publication of application: 10.03.1993
(73) Proprietor: KIMBERLY-CLARK WORLDWIDE, INC., Neenah, Wisconsin 54956 (US)
(72) Inventor: Tsai, Chuan-Ling, Appleton, Wisconsin 54915 (US)
(74) Representative: Diehl, Hermann O. Th., Dr.

(56) References cited:
- EP-A- 0 295 438
- WO-A-91/02552
- US-A- 4 920 202
- US-A- 5 075 344

## Description

This invention relates to a method of treating water-insoluble superabsorbent materials.

Superabsorbent materials are well known materials which have particular utility in absorbent personal products such as disposable diapers, training pants, incontinence garments and feminine pads. As their name suggests, the purpose of superabsorbents is to absorb large quantities of liquid in amounts many times the weight of the superabsorbent material. One difficulty with many superabsorbent materials, however, is that the in-use absorbent capacity is less than that measured in the laboratory due to pressure or loading experienced under actual in-use conditions. In the case of a disposable diaper, for example, when the baby is sitting down, the weight of the baby causes increased pressure to be exerted on the superabsorbent, thereby decreasing its ability to absorb and hold liquid. To this end, there is continual effort to provide superabsorbent materials which have increased absorbent capacity in use.

Document WO-A-91 02552 discloses a superabsorbent material whose original structure has been modified by expansion, such that the final structure of the material is expanded. One embodiment of the material is produced using a thermo treatment.

In order to solve this object, the invention provides a method of treating water-insoluble superabsorbent materials according to independent claim 1. Further advantageous features, aspects and details are evident from the dependent claims, the description, examples and tables.

The invention by means of thermal treatment enhances the rate of absorbency under load of superabsorbent materials.

The invention is based on the discovery that the in-use absorbent capacity of superabsorbent materials, when measured under a load, can be improved by simply heating the superabsorbent material for a period of time at a sufficiently high temperature. This property of the superabsorbent material is measured by the Absorbency Under Load (AUL) test, which will be hereinafter described with reference to the drawing. Interestingly, the heat treatment of this invention has shown no positive effect on the free swell absorbent capacity of superabsorbent materials as normally measured without an applied load when using the same saline solution used to determine the AUL. The temperature/time relationship of the heat treatment of this invention must be optimized for each particular superabsorbent material, but in general, higher temperatures and longer times improve the AUL. Hence high temperature for a short treatment time can produce good results as well as low temperature for a long treatment time. In carrying out the method of this invention, the 2-minute AUL of the superabsorbent material is preferably increased about 1 gram per gram or greater.

Treatment temperatures from about 140°C. to about 210°C. have been shown to work well, although lower and higher temperatures can be used. A temperature of 125°C. is considered to be the lower limit for practical purposes due to the correspondingly long time normally necessary to obtain sufficiently improved results. On the other hand, the upper treatment temperature is limited only by the ability of the superabsorbent material to withstand the temperature treatment without degrading or melting. For polyacrylate superabsorbent materials, for example, an upper temperature limit is about 350°C. For commercial purposes, the highest possible temperatures are preferred because of the attendant short treatment times. It is not necessary that the treatment temperature be held constant during the treatment. Accordingly the temperature can increase, decrease, or cycle up and down within the selected range.

The treatment time is preferably as short as possible, provided the 2 minute AUL of the superabsorbent material is increased at least about 1 gram per gram. When using the preheated forced air oven, however, treatment times are necessarily about 1 or 2 minutes or longer due to heat transfer limitations. Treatment times of from 5 minutes to 60 minutes have been used successfully, with a treatment time of 20 minutes or less being preferred, more preferably 10 minutes or less, and most preferably about 5 minutes. As stated above, the treatment time will be dependent on the temperature of the treatment.

Suitable superabsorbent materials include any substantially water-insoluble material which is capable of absorbing or gelling at least 10 times its weight, preferably 15 times its weight, of body exudate or a suitable aqueous solution such as a 0.9 weight percent solution of sodium chloride in distilled water. Such materials can include, but are not limited to, synthetic materials and modified natural materials. Preferably the superabsorbent materiais are once dried, as commercially received from the superabsorbent manufacturer (up to 7 weight percent moisture), prior to being subjected to the heat treatment of this invention. However, the heat treatment of this invention can also be applied during the initial drying or the superabsorbent material after it is made. By way of example, superabsorbent materials include, hydrogel-forming polymers which are alkali metal salts of: poly(acrylic acid); poly(methacrylic acid); copolymers of acrylic and methacrylic acid with acrylamide, vinyl alcohol, acrylic esters, vinyl pyrrolidone, vinyl sulfonic acids, vinyl acetate, vinyl morpholinone and vinyl ethers; hydrolyzed acrylonitrile grafted starch; acrylic acid grafted starch; maleic anhydride copolymers with ethylene, isobutylene, styrene, and vinyl ethers; polysaccharides such as carboxymethyl starch, carboxymethyl cellulose, methyl cellulose, and hydroxypropyl cellulose; poly(acrylamides); poly(vinyl pyrrolidone); poly(vinyl morpholinone); poly(vinyl pyridine); and copolymers and mixtures of any of the above. The hydrogel-forming polymers are preferably lightly crosslinked to render them substantially water-insoluble. A preferable superabsorbent material is a lightly crosslinked poly(sodium acrylate). Crosslinking may be achieved by irradiation or by covalent, ionic, van der Waals attractions, or hydrogen bonding interactions, for example. The superabsorbent materials can be in any form suitable for use in absorbent structures, including particles, fibers, bicomponent fibers, filaments, flakes and spheres.

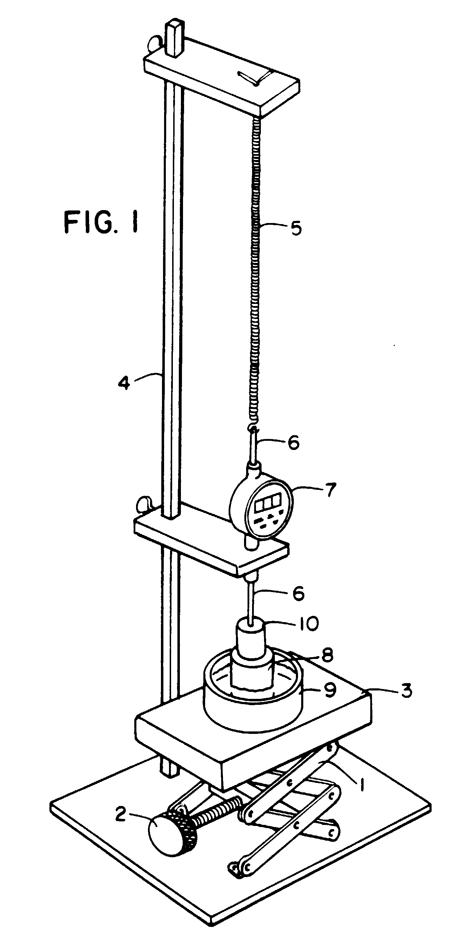

Figure 1, the sole Figure in the drawing, is a schematic illustration of the apparatus used for measuring the Absorbency Under Load of a superabsorbent material.

The Absorbency Under Load (AUL) is a test which measures the ability of a superabsorbent material to absorb a liquid (0.9 weight percent solution of sodium chloride in distilled water) while under an applied load or restraining force.

Referring to Figure 1, the apparatus and method for determining AUL will be described. Shown is a perspective view of the apparatus in position during a test. Shown is a laboratory jack 1 having an adjustable knob 2 for raising and lowering the platform 3. A laboratory stand 4 supports a spring 5 connected to a modified thickness meter probe 6, which passes through the housing 7 of the meter, which is rigidly supported by the laboratory stand. A plastic sample cup 8, which contains the superabsorbent material sample to be tested, has a liquid-permeable bottom and rests within a Petri dish 9, which contains the saline solution to be absorbed. A weight 10 rests on top of a spacer disc (not visible) resting on top of the superabsorbent material sample (not visible).

The sample cup consists of a plastic cylinder having a 2.54 cm (1 inch) inside diameter and an outside diameter of 3.18 cm (1.25 inch). The bottom of the sample cup is formed by adhering a 300 µm mesh metal screen to the end of the cylinder by heating the screen above the melting point of the plastic and pressing the plastic cylinder against the hot screen to melt the plastic and bond the screen to the plastic cylinder.

The modified thickness meter used to measure the expansion of the sample while absorbing the saline solution is a Mitutoyo Digimatic Indicator, IDC Series 543, Model 543-180, having a range of 0-1.3 cm (0-0.5 inch) and an accuracy of 0.00013 cm (0.00005 inch) (Mitutoyo Corporation, 31-19, Shiba 5-chome, Minato-ku, Tokyo 108, Japan). As supplied from Mitutoyo Corporation, the thickness meter contains a spring attached to the probe within the meter housing. This spring is removed to provide a free falling probe, which has a downward force of about 27 grams. In addition, the cap over the top of the probe located on the top of the meter housing is also removed to enable attachment of the probe to the suspension spring 5 (available from McMaster-Carr Supply Co., Chicago, Illinois, Item No.9640K41), which serves to counter or reduce the downward force of the probe to about 1 gram, ±0.5 gram. A wire hook can be glued to the top of the probe for attachment to the suspension spring. The bottom tip of the probe is also provided with an extension needle (Mitutoyo Corporation, Part No. 131279) to enable the probe to be inserted into the sample cup.

To carry out the test, a 0.160 gram sample of the superabsorbent material, which has been sieved to a particle size between 300 and 600 µm, is placed into the sample cup. The sample is then covered with a plastic spacer disc, weighing 4.4 grams, which is slightly smaller than the inside diameter of the sample cup and serves to protect the sample from being disturbed during the test. The 100 grams weight is then placed on top of the spacer disc, thereby applying a load of 0.021 kg/cm² (0.3 pounds per square inch). The sample cup is placed in the Petri dish on the platform of the laboratory jack raised up until it contacts the tip of the probe. The meter is zeroed. A sufficient amount of saline solution is added to the Petri dish (50-100 milliliters) to begin the test. The distance the weight is raised by the expanding sample as it absorbs the saline solution is measured by the probe. This distance, multiplied by the cross-sectional area inside the sample cup, is a measure of the expansion volume of the sample due to absorption. Factoring in the density of the saline solution and the weight of the sample, the amount of saline solution absorbed is readily calculated. The weight of saline solution absorbed after 2, 4, 10 or 30 minutes is the AUL value for that length of time, expressed as grams saline solution absorbed per gram of superabsorbent. If desired, the readings of the modified thickness meter can be continuously input to a computer (Mitutoyo Digimatic Miniprocessor DP-2 DX) to make the calculations and provide AUL readings. As a cross-check, the AUL can also be determined by determining the weight difference between the sample cup before and after the test, the weight difference being the amount of solution absorbed by the sample.

In order to illustrate the method of this invention, several superabsorbent materials were subjected to different time/temperature treatments. Specifically, a 10 gram once-dried sample of the superabsorbent material as received from the superabsorbent manufacturer, which had been sieved to a particle size in the range of from 300 to 600 µm, was placed in a glass beaker and thereafter placed in a preheated forced air oven (B-2730-Q, Blue M, Blue Island, Illinois) for a fixed length of time. The sample was removed from the oven and allowed to cool to ambient temperature. The AUL of the sample, at 0.021 kg/cm² (0.3 pounds per square inch) pressure, was then measured at 2 minutes, 4 minutes, 10 minutes and 30 minutes.

The particular superabsorbents tested were: IM-5000P (Starch grafted sodium polyacrylate, Hoechst Celanese Corporation, Portsmouth, Virginia); DRYTECH 534 (Partial sodium salt of crosslinked poly(acrylic acid), Dow Chemical Company, Midland, Michigan); FAVOR SAB 835 (Polyacrylate/polyalcohol, Stockhausen, Inc., Greensboro, North Carolina); 88-103 (Partial sodium salt of crosslinked poly(acrylic acid), Dow Chemical Company); 88-111 (Partial sodium salt of crosslinked poly(acrylic acid), Dow Chemical Company); KI Gel (isobutylene/maleic anhydride copolymer, Kuraray Co., Ltd., Tokyo, Japan; FOXORB HR (carboxymethylated starch, Avebe, Foxhol, Netherlands); AQUALON (crosslinked carboxymethylated cellulose, (Aqualon Company, Wilmington, Delaware).

The results are tabulated in TABLES 1 and 2, in which the effects of treatment time and temperature, respectively, are illustrated.

**TABLE 1**

| (Effect of Treatment Time on AUL) | | | | |
|---|---|---|---|---|
| SAMPLE | 2 min AUL | 4 min AUL | 10 min AUL | 30 min AUL |
| IM-5000P | | | | |
| Untreated | 2.75 | 4.05 | 8.96 | 24.84 |
| 170°C/5 min. | 3.92 | 5.86 | 11.17 | 26.20 |
| 170°C/10 min. | 3.78 | 5.80 | 13.14 | 27.26 |
| 170°C/15 min. | 12.44 | 20.23 | 26.08 | 27.32 |
| 170°C/20 min. | 11.43 | 18.65 | 25.19 | 26.61 |
| 170°C/30 min. | 13.32 | 20.64 | 25.11 | 25.80 |
| 170°C/60 min. | 14.27 | 20.35 | 23.35 | 23.60 |
| | | | | |

| DRYTECH 534 | | | | |
|---|---|---|---|---|
| Untreated | 4.06 | 6.25 | 11.67 | 21.49 |
| 180°C/15 min. | 5.08 | 10.63 | 19.30 | 23.94 |
| 180°C/20 min. | 6.43 | 12.34 | 20.76 | 24.68 |
| 180°C/25 min. | 6.18 | 12.13 | 20.33 | 24.08 |
| 180°C/30 min. | 6.37 | 12.48 | 20.76 | 24.48 |
| | | | | |

| FAVOR SAB 835 | | | | |
|---|---|---|---|---|
| Untreated | 3.88 | 5.15 | 8.77 | 22.42 |
| 190°C/10 min. | 5.46 | 8.76 | 19.80 | 25.08 |
| 190°C/15 min. | 8.03 | 14.99 | 22.72 | 25.61 |
| 190°C/20 min. | 10.36 | 17.64 | 23.84 | 25.80 |
| 190°C/25 min. | 10.48 | 17.89 | 24.34 | 26.29 |
| 190°C/30 min. | 9.68 | 17.07 | 23.85 | 26.12 |

**TABLE 2**

| (Effect of Treatment Temperature on AUL) | | | | |
|---|---|---|---|---|
| SAMPLE | 2 min AUL | 4 min AUL | 10 min AUL | 30 min AUL |
| IM-5000P | | | | |
| Untreated | 2.75 | 4.05 | 8.96 | 24.84 |
| 100°C/20 min. | 2.62 | 3.75 | 6.95 | 21.90 |
| 140°C/20 min. | 3.15 | 4.73 | 10.62 | 26.36 |
| 150°C/20 min. | 4.60 | 7.26 | 15.66 | 27.44 |
| 160°C/20 min. | 6.90 | 13.46 | 23.84 | 27.37 |
| 170°C/20 min. | 9.27 | 17.69 | 24.87 | 26.42 |
| 180°C/20 min. | 12.33 | 19.81 | 24.13 | 24.84 |
| 190°C/20 min. | 12.37 | 18.92 | 21.97 | 22.32 |
| 200°C/20 min. | 13.06 | 18.54 | 20.58 | 20.73 |
| | | | | |

| DRYTECH 534 | | | | |
|---|---|---|---|---|
| Untreated | 4.06 | 6.25 | 11.67 | 21.49 |
| 170°C/20 min. | 5.78 | 10.71 | 19.47 | 23.97 |
| 180°C/20 min. | 6.43 | 12.34 | 20.76 | 24.68 |
| 190°C/20 min. | 5.09 | 10.95 | 19.39 | 23.32 |
| 200°C/20 min. | 6.47 | 12.78 | 20.61 | 23.69 |

| FAVOR SAB 835 | | | | |
|---|---|---|---|---|
| Untreated | 3.88 | 5.15 | 8.77 | 22.42 |
| 170°C/20 min. | 4.44 | 5.91 | 11.78 | 25.19 |
| 180°C/20 min. | 7.14 | 12.97 | 22.64 | 25.98 |
| 190°C/20 min. | 10.36 | 17.64 | 23.84 | 25.80 |
| 200°C/20 min. | 9.60 | 16.69 | 23.53 | 25.91 |
| | | | | |

| 88-103 | | | | |
|---|---|---|---|---|
| Untreated | 9.67 | 16.09 | 22.47 | 24.51 |
| 180°C/20 min. | 9.84 | 16.77 | 22.85 | 24.52 |
| 190°C/20 min. | 12.31 | 19.31 | 24.41 | 25.80 |
| 200°C/20 min. | 13.12 | 20.61 | 24.24 | 24.85 |
| 210°C/20 min. | 13.91 | 20.76 | 22.97 | 23.17 |
| | | | | |

| 88-111 | | | | |
|---|---|---|---|---|
| Untreated | 13.61 | 20.96 | 25.50 | 26.53 |
| 180°C/20 min. | 15.34 | 20.94 | 24.21 | 24.92 |
| 190°C/20 min. | 17.15 | 21.84 | 24.23 | 24.75 |
| 200°C/20 min. | 19.00 | 22.41 | 23.82 | 23.99 |
| 210°C/20 min. | 19.26 | 21.75 | 22.36 | 22.43 |
| | | | | |

| KI Gel | | | | |
|---|---|---|---|---|
| Untreated | 2.43 | 3.45 | 5.19 | 6.91 |
| 180°C/20 min. | 3.46 | 4.74 | 6.84 | 8.80 |
| 200°C/20 min. | 4.35 | 6.00 | 8.31 | 10.25 |

| FOXORB HR | | | | |
|---|---|---|---|---|
| Untreated | 2.62 | 3.28 | 4.17 | 5.26 |
| 110°C/20 min. | 2.50 | 3.28 | 4.44 | 5.86 |
| 170°C/20 min. | 11.21 | 11.26 | 11.30 | 11.35 |
| | | | | |

| AQUALON | | | | |
|---|---|---|---|---|
| Untreated | 1.22 | 1.54 | 2.13 | 3.23 |
| 170°C/20 min. | 2.39 | 2.78 | 3.39 | 4.38 |

The results of these tests illustrate the improvement in AUL which is achieved using the heat treatment of this invention. In all cases, the 2 minute AUL was increased after the superabsorbent material was heat treated at temperatures of 140°C. or greater. Similarly, the 2 minute AUL was increased after being treated for 5 minutes or more for all samples tested.

## Claims

1. A method of treating a once-dried water-insoluble superabsorbent material having up to 7 weight percent moisture, comprising heating the once-dried water-insoluble superabsorbent material in a preheated forced air oven at a temperature of 125°C or greater for a time of from 5 minutes to 60 minutes to increase the 2-minute Absorbency Under Load of the superabsorbent material, which is determined under an applied load of 0.021 kg/cm² (0.3 pounds per square inch), at least 1 gram per gram.

2. The method of Claim 1 wherein the temperature is 140°C. or greater.

3. The method of Claim 1 wherein the temperature is 150°C. or greater.

4. The method of Claim 1 wherein the temperature is 160°C. or greater.

5. The method of Claim 1 wherein the temperature is 170°C. or greater.

6. The method of Claim 1 wherein the temperature is 180°C. or greater.

7. The method of Claim 1 wherein the temperature is 190°C. or greater.

8. The method of Claim 1 wherein the temperature is 200°C. or greater.

9. The method of Claim 1 wherein the temperature is 210°C. or greater.

10. The method of Claim 1 wherein the temperature is from 150°C. to 210°C.

11. The method of any one of the preceding claims wherein the superabsorbent material is treated for 5 minutes.

12. The method of any one of Claims 1 to 10 wherein the superabsorbent material is treated for 10 minutes or less.

13. The method of any one of Claims 1 to 10 wherein the superabsorbent material is treated for 20 minutes or less.

14. The method of any one of Claims 1 to 10 wherein the superabsorbent material is treated for 25 minutes or less.

15. The method of any one of Claims 1 to 10 wherein the superabsorbent material is treated for 30 minutes or less.

16. The method of any one of Claims 1 to 10 wherein the superabsorbent material is treated for 60 minutes or less.

17. The method of Claim 1 wherein the superabsorbent material is treated at a temperature of from 140°C. to 210°C. for from 5 to 60 minutes.

18. The method of Claim 1 wherein the superabsorbent material is treated at a temperature of from 170°C. to 210°C. for 20 minutes or less.

19. The method of Claim 1 wherein the superabsorbent material is treated at a temperature of from 170°C. to 190°C. for 20 minutes or less.

20. The method of any one of the preceding claims wherein the superabsorbent material is an alkali metal salt of poly(acrylic acid).

## Patentansprüche

1. Verfahren zur Behandlung eines einmal getrockneten wasserunlöslichen Superabsorptionsmaterials mit bis zu 7 Gew.% Feuchtigkeit, umfassend eine Erwärmung des einmal getrockneten wasserunlöslichen Superabsorptionsmaterials in einem vorgewärmten Gebläseluftofen bei einer Temperatur von 125°C oder höher über eine Zeit von 5 Minuten bis 60 Minuten zur Erhöhung des 2-Minuten-Absorptionsvermögens unter Belastung des Superabsorptionsmaterials, welches unter einer angewendeten Belastung von 0,021 kg/cm² (0,3 Pounds pro Quadratinch) bestimmt wird, um mindestens 1 Gramm pro Gramm.

2. Verfahren gemäß Anspruch 1, bei dem die Temperatur 140°C oder mehr beträgt.

3. Verfahren gemäß Anspruch 1, bei dem die Temperatur 150°C oder mehr beträgt.

4. Verfahren gemäß Anspruch 1, bei dem die Temperatur 160°C oder mehr beträgt.

5. Verfahren gemäß Anspruch 1, bei dem die Temperatur 170°C oder mehr beträgt.

6. Verfahren gemäß Anspruch 1, bei dem die Temperatur 180°C oder mehr beträgt.

7. Verfahren gemäß Anspruch 1, bei dem die Temperatur 190°C oder mehr beträgt.

8. Verfahren gemäß Anspruch 1, bei dem die Temperatur 200°C oder mehr beträgt.

9. Verfahren gemäß Anspruch 1, bei dem die Temperatur 210°C oder mehr beträgt.

10. Verfahren gemäß Anspruch 1, bei dem die Temperatur 150°C bis 210°C beträgt.

11. Verfahren gemäß einem der vorhergehenden Ansprüche, bei dem das Superabsorptionsmaterial 5 Minuten lang behandelt wird.

12. Verfahren gemäß einem der Ansprüche 1 bis 10, bei dem das Superabsorptionsmaterial 10 Minuten oder weniger behandelt wird.

13. Verfahren gemäß einem der Ansprüche 1 bis 10, bei dem das Superabsorptionsmaterial 20 Minuten oder weniger behandelt wird.

14. Verfahren gemäß einem der Ansprüche 1 bis 10, bei dem das Superabsorptionsmaterial 25 Minuten oder weniger behandelt wird.

15. Verfahren gemäß einem der Ansprüche 1 bis 10, bei dem das Superabsorptionsmaterial 30 Minuten oder weniger behandelt wird.

16. Verfahren gemäß einem der Ansprüche 1 bis 10, bei dem das Superabsorptionsmaterial 60 Minuten oder weniger behandelt wird.

17. Verfahren gemäß Anspruch 1, bei dem das Superabsorptionsmaterial bei einer Temperatur von 140°C bis 210°C 5 bis 60 Minuten behandelt wird.

18. Verfahren gemäß Anspruch 1, bei dem das Superabsorptionsmaterial bei einer Temperatur von 170°C bis 210°C 20 Minuten oder weniger behandelt wird.

19. Verfahren gemäß Anspruch 1, bei dem das Superabsorptionsmaterial bei einer Temperatur von 170°C bis 190°C 20 Minuten oder weniger behandelt wird.

20. Verfahren gemäß einem der vorhergehenden Ansprüche, bei dem das Superabsorptionsmaterial ein Alkalimetallsalz von Poly(acrylsäure) ist.

## Revendications

1. Procédé de traitement d'un matériau superabsorbant insoluble dans l'eau une fois séché, lequel renferme jusqu'à 7 % en poids d'humidité, comprenant le chauffage du matériau superabsorbant, insoluble dans l'eau une fois séché, dans un four à circulation d'air forcée préchauffé, à une température de 125°C ou plus, pendant une période de temps comprise entre 5 minutes et 60 minutes, pour augmenter la Capacité d'Absorption sous Charge en 2 minutes du matériau superabsorbant, laquelle est déterminée sous une charge appliquée de 0,021 kg/cm² (0,3 livre/pouce²), d'au moins 1 gramme par gramme.

2. Procédé selon la revendication 1, dans lequel la température est de 140°C ou plus.

3. Procédé selon la revendication 1, dans lequel la température est de 150°C ou plus.

4. Procédé selon la revendication 1, dans lequel la température est de 160°C ou plus.

5. Procédé selon la revendication 1, dans lequel la température est de 170°C ou plus.

6. Procédé selon la revendication 1, dans lequel la température est de 180°C ou plus.

7. Procédé selon la revendication 1, dans lequel la température est de 190°C ou plus.

8. Procédé selon la revendication 1, dans lequel la température est de 200°C ou plus.

9. Procédé selon la revendication 1, dans lequel la température est de 210°C ou plus.

10. Procédé selon la revendication 1, dans lequel la température est comprise entre 150 et 210°C.

11. Procédé selon l'une quelconque des revendications précédentes, dans lequel le matériau superabsorbant est traité pendant 5 minutes.

12. Procédé selon l'une quelconque des revendications 1 à 10, dans lequel le matériau superabsorbant est traité pendant 10 minutes ou moins.

13. Procédé selon l'une quelconque des revendications 1 à 10, dans lequel le matériau superabsorbant est traité pendant 20 minutes ou moins.

14. Procédé selon l'une quelconque des revendications 1 à 10, dans lequel le matériau superabsorbant est traité pendant 25 minutes ou moins.

15. Procédé selon l'une quelconque des revendications 1 à 10, dans lequel le matériau superabsorbant est traité pendant 30 minutes ou moins.

16. Procédé selon l'une quelconque des revendications 1 à 10, dans lequel le matériau superabsorbant est traité pendant 60 minutes ou moins.

17. Procédé selon la revendication 1, dans lequel le matériau superabsorbant est traité à une température comprise entre 140°C et 210°C pendant 5 à 60 minutes.

18. Procédé selon la revendication 1, dans lequel le matériau superabsorbant est traité à une température comprise entre 170°C et 210°C, pendant 20 minutes ou moins.

19. Procédé selon la revendication 1, dans lequel le matériau superabsorbant est traité à une température comprise entre 170°C et 190°C, pendant 20 minutes ou moins.

20. Procédé selon l'une quelconque des revendications précédentes, dans lequel le matériau superabsorbant est un sel de métal alcalin d'un poly(acide acrylique).
